(19) **Europäisches Patentamt / European Patent Office / Office européen des brevets**

(11) **EP 3 955 832 B1**

(12) **EUROPEAN PATENT SPECIFICATION**

(45) Date of publication and mention
of the grant of the patent:
**01.07.2026 Bulletin 2026/27**

(21) Application number: **20723602.7**

(22) Date of filing: **01.04.2020**

(51) International Patent Classification (IPC):
**A61B 17/11** *(2006.01)* **A61F 2/07** *(2013.01)*

(52) Cooperative Patent Classification (CPC):
**A61B 17/11; A61F 2/07; A61F 2/90;**
A61B 2017/00358; A61B 2017/1107;
A61B 2017/1135; A61B 2017/1139; A61F 2/856;
A61F 2/962; A61F 2002/061; A61F 2210/0076;
A61F 2230/001; A61F 2230/0065; A61F 2250/001;
A61F 2250/0039

(86) International application number:
**PCT/US2020/026094**

(87) International publication number:
**WO 2020/214416 (22.10.2020 Gazette 2020/43)**

(54) **TRANSCAVAL VENTURI**

TRANSKAVALER VENTURI

VENTURI TRANSCAVITAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.04.2019 US 201962835610 P**

(43) Date of publication of application:
**23.02.2022 Bulletin 2022/08**

(73) Proprietor: **Edwards Lifesciences Corporation
Irvine, CA 92614 (US)**

(72) Inventors:
• **MANASH, Boaz**
**3052602 Givat Ada (IL)**
• **GOLDBERG, Eran**
**30889 Caesarea (IL)**

(74) Representative: **Eisenführ Speiser
Patentanwälte Rechtsanwälte PartGmbB
Postfach 31 02 60
80102 München (DE)**

(56) References cited:
WO-A2-2015/013344      US-A1- 2003 024 527
US-A1- 2006 287 704      US-A1- 2013 261 531

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND

<u>Field</u>

The present disclosure relates to the field of medical devices and procedures.

<u>Description of the Related Art</u>

Congestive heart failure (CHF) affects large numbers of people. In some congestive heart failure cases, there may be systemic fluid congestion, such as that resulting from right-sided heart failure. This may lead to shortness of breath, fatigue, and eventually hospitalization.

Shunts between blood vessels are used to provide blood flow between vessels and used to treat various conditions. One aspect of such shunts is the flow of blood therethrough. However, too little blood flow may fail to provide the desired treatment results, and too much blood flow may create other complications.

US 2003/024527 A1 relates to a lung-assist apparatus which includes a tubular housing, a tubular nozzle therein, and a first valve disposed between the housing and nozzle. The housing is implanted across a bifurcation such that the nozzle extends from a first branch communicating with a healthy region of a lung towards a main passage, and terminates proximate a lateral opening in the housing that is disposed within a second branch communicating with a damaged region of the lung. During inhalation, the first valve opens to allow air flow into the first branch, and closes during exhalation to force air through the nozzle, thereby inducing a vacuum for drawing air from the damaged region. A second valve in the second branch opens during exhalation to draw air from the diseased region, and closes during inhalation to prevent air from being drawn into the damaged region.

Further, US 2006/287704 A1 relates to a side branch stent graft suitable for the iliac arteries which has a main tubular body of a biocompatible graft material and a tubular side branch. The tubular side branch is affixed into the main tubular body so that a side branch lumen is in fluid communication with a main lumen. There is at least one external zig-zag stent on the main tubular body proximal of the tubular side branch, one central external stent which also encompasses the side arm, at least one external zig-zag stent on the main tubular body distal of the tubular side branch and one internal zig-zag stent at the distal end of the main tubular body. A reinforcing ring is around the proximal end of the main tubular body and stitched thereto.

WO 2015/013344 A2 relates to vascular graft suitable for implantation, and more particularly to a vascular graft having an expandable outflow region for restoring patency of the graft after implantation into a body lumen.

SUMMARY

The present disclosure describes systems, devices, and methods for providing fluid connections between blood vessels, such as may be desired to treat congestive heart failure and other conditions.

The scope of the claimed invention is defined by independent claim 1. The dependent claims define preferred embodiments of the claimed invention.

In some implementations, the present disclosure relates to a device for providing fluid flow between fluid vessels of a patient. The device comprises a conduit structure configured to be implanted within a first fluid vessel, the conduit structure comprising an outer wall defining a conduit lumen extending between a conduit inlet and a conduit outlet, and the conduit structure comprising a constricted portion positioned between the conduit inlet and the conduit outlet. The constricted portion comprising a constricted lumen diameter that is less than a diameter of at least one of the conduit inlet or the conduit outlet. The device also comprises a shunt structure configured to be at least partially implanted within a second fluid vessel that is adjacent to the first fluid vessel and configured to attach to the constricted portion of the conduit structure. The shunt structure comprising a shunt lumen that is configured to be in fluid communication with the conduit lumen.

The shunt lumen can comprise a shunt lumen diameter that is no greater than the constricted lumen diameter. For example, the shunt lumen diameter can be 0.8 or less of the constricted lumen diameter. In some embodiments, the shunt structure can comprise a shunt anchor configured to be secured to at least a portion of the second fluid vessel. The shunt anchor can comprise a stent adapted to be radially deployed within the second fluid vessel and placed into contact with a wall of the second fluid vessel. The first fluid vessel can be an abdominal aorta and the second fluid vessel can be an inferior vena cava.

The conduit structure can comprise a self-expanding stent frame configured to expand radially to engage a wall of the first fluid vessel. For example, the self-expanding stent frame can have a delivery configuration and an expanded configuration, the self-expanding stent frame being biased toward the expanded configuration. In some embodiments, the conduit structure and the shunt structure can each comprise a self-expanding structure. The conduit structure and the shunt structure can each comprise memory material and are biased toward an expanded configuration. In some embodiments, the conduit structure can comprise a bifurcation between the constricted portion and the conduit outlet, and the conduit structure can comprise an outlet downstream of the bifurcation.

In some embodiments, the diameter of the conduit outlet can be the same as the constricted lumen diameter. Further, in some embodiments, the diameter of the conduit outlet can be larger than the constricted lumen diameter. The constricted lumen diameter can be less than 0.8 of the diameter of the conduit inlet.

In some implementations, the present disclosure relates to a system for providing fluid flow between fluid vessels of a patient. The system comprises a conduit structure configured to be implanted within a first fluid vessel, the conduit structure comprising a conduit lumen extending between a first end of the conduit structure and a second end of the conduit structure, and the conduit structure comprising a narrowed portion disposed between the first end and the second end. A diameter of the narrowed portion is less than at least one of a diameter of the first end or a diameter of the second end. The system also comprises a shunt structure attached to the narrowed portion of the conduit structure and configured to be at least partly disposed in a second fluid vessel that is adjacent to the first fluid vessel. The conduit structure is configured to draw fluid from the second fluid vessel into the first fluid vessel through the shunt structure.

The system can further comprise a delivery catheter to removably receive the conduit structure and the shunt structure and configured to hold at least one of the conduit structure or the shunt structure in a compressed delivery configuration. The conduit structure can be self-expanding, and the delivery catheter can comprise a sheath configured to slidingly receive the conduit structure. The delivery catheter can comprise a radially expandable balloon configured to deploy the conduit structure within the first fluid vessel. The delivery catheter can be configured to deploy the shunt structure between the first fluid vessel and the second fluid vessel.

The shunt structure can comprise a shunt anchor configured to secure at least a portion of the shunt structure against a wall of the second fluid vessel. For example, the shunt anchor can comprise a stent configured to be radially deployed within the second fluid vessel. The shunt anchor can comprise a disk-shaped form. In some embodiments, the first fluid vessel can be an aorta and the second fluid vessel can be an inferior vena cava. Further, in some embodiments, the diameter of the narrowed portion of the conduit structure can be adjustable.

In some implementations, the present disclosure relates to a method of providing a fluid connection between fluid vessels. The method comprises advancing a delivery catheter to a target location, the delivery catheter including a conduit structure and a shunt structure attached thereon, advancing the delivery catheter into anatomy between a first fluid vessel and a second fluid vessel that is adjacent to the first fluid vessel, deploying the conduit structure within the second fluid vessel, the conduit structure including a first section, a second section, and a lumen extending through the first section and the second section, a diameter of the second section being smaller than a diameter of the first section, and deploying the shunt structure in the anatomy between the first fluid vessel and the second fluid vessel with the shunt structure extending into the first fluid vessel and being connected to the second section of the conduit structure.

The advancing the delivery catheter to the target location can comprise advancing the delivery catheter within the first fluid vessel. In some embodiments, the method can further comprise creating a passage within the anatomy between the first fluid vessel and the second fluid vessel. The advancing the delivery catheter into the anatomy can comprise advancing the delivery catheter into the passage. The deploying the shunt structure in the anatomy between the first fluid vessel and the second fluid vessel can comprise sealing the passage such that fluid flow through the passage is prevented except for fluid flow through the shunt structure.

The deploying the shunt structure in the anatomy between the first fluid vessel and the second fluid vessel can occur after the deploying the conduit structure within the second fluid vessel. In some embodiments, the shunt structure comprises an anchor, and the method further comprises deploying the anchor within the first fluid vessel. The deploying the anchor within the first fluid vessel can occur after the deploying the shunt structure in the anatomy between the first fluid vessel and the second fluid vessel. The first fluid vessel can be an inferior vena cava and the second fluid vessel can be an aorta.

The advancing the delivery catheter to the target location can comprise advancing the delivery catheter through a femoral vein and through the inferior vena cava. In some embodiments, the method can further comprise securing the shunt structure to the delivery catheter prior to advancing the delivery catheter to the target location. Further, in some embodiments, the method can further comprise adjusting the diameter of the second section of the conduit structure upon deploying the conduit structure within the second fluid vessel. The adjusting the diameter of the second section of the conduit structure can comprise using a tensioning line that extends through the conduit structure to reduce the diameter of the second section of the conduit structure.

The deploying the conduit structure within the second fluid vessel can comprise radially expanding the conduit structure, and the deploying the shunt structure in the anatomy between the first fluid vessel and the second fluid vessel can comprise radially expanding the shunt structure. In some embodiments, the conduit structure and the shunt structure are each self-expanding. Further, in some embodiments, the delivery catheter comprises a sheath, the deploying the conduit structure within the second fluid vessel comprises slidingly retracting the sheath with respect to the conduit structure, and the deploying the shunt structure in the anatomy between the first fluid vessel and the second fluid vessel comprises slidingly retracting the sheath with respect to the shunt structure.

It should be understood that each of the elements disclosed herein can be used with any and all of the elements disclosed herein, even though the specific combination of elements may not be explicitly shown in the figures herein. In other words, based on the explanation of the particular device, one of skill in the art should have little trouble combining the features of certain of two such devices. Therefore, it should be understood that many of the elements are interchangeable, and the disclosure covers all permutations thereof.

Other objects, features, and advantages of the present disclosure will become apparent from a consideration of the following detailed description.

BRIEF DESCRIPTION OF THE DRAWINGS

FIG. 1 is a side view of an example device deployed in a patient according to one or more embodiments;

FIGS. 2A-2D depict side, first end, bottom, and second end views of an example device according to one or more embodiments;

FIG. 3 depicts a diagrammatic image of an example stent portion according to one or more embodiments;

FIG. 4-1 depicts a front view of an example device having a single funnel shaped portion according to one or more embodiments;

FIG. 4-2 depicts the device of FIG. 4-1 deployed in a patient according to one or more embodiments;

FIG. 5 depicts a front view, in partial cross-section, of kidneys with treatment according to one or more embodiments;

FIG. 6 depicts a side view of an example device according to one or more embodiments;

FIGS. 7A-7C depict side views, in cross-section, of a stent-like frame portion according to one or more embodiments;

FIGS. 8A-8C depict end, perspective, and side views, respectively, of an example stent-like frame portion according to one or more embodiments;

FIG. 9 depicts a side view of an example device according to one or more embodiments;

FIG. 10 depicts a side view of an example device according to one or more embodiments;

FIGS. 11A-11D depict diagrammatic side views, in partial cross-section, of an example device with a delivery catheter according one or more embodiments;

FIGS. 12A-12D depict front views of a patient's anatomy during delivery and deployment of an example device according to one or more embodiments;

FIG. 13 depicts an example process for implanting a device within one or more vessels of a patient according to one or more embodiments; and

FIG. 14 depicts an example device with an anchor implemented as a stent according to one or more embodiments.

DETAILED DESCRIPTION

Although certain embodiments and examples are disclosed below, the subject matter extends beyond the specifically disclosed embodiments to other alternative embodiments and uses, and to modifications and equivalents thereof. Thus, the scope of the claims that may arise herefrom is not limited by any of the particular embodiments described herein. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent. Additionally, the structures, systems, and devices described herein may be embodied as integrated components or as separate components. For purposes of comparing various embodiments, certain aspects and advantages of these embodiments are described. Not necessarily all of such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, embedded at least partially within, or otherwise physically related to the second feature, element, component, device, or member, whether directly or indirectly.

The systems, devices, and methods of the present disclosure can be utilized in various surgical procedures as well as minimally invasive procedures, percutaneous procedures, and other procedures. In some embodiments, the methods, systems, and devices can be delivered and deployed via a catheter or catheters into the patient's arterial system (e.g., through the femoral or brachial arteries).

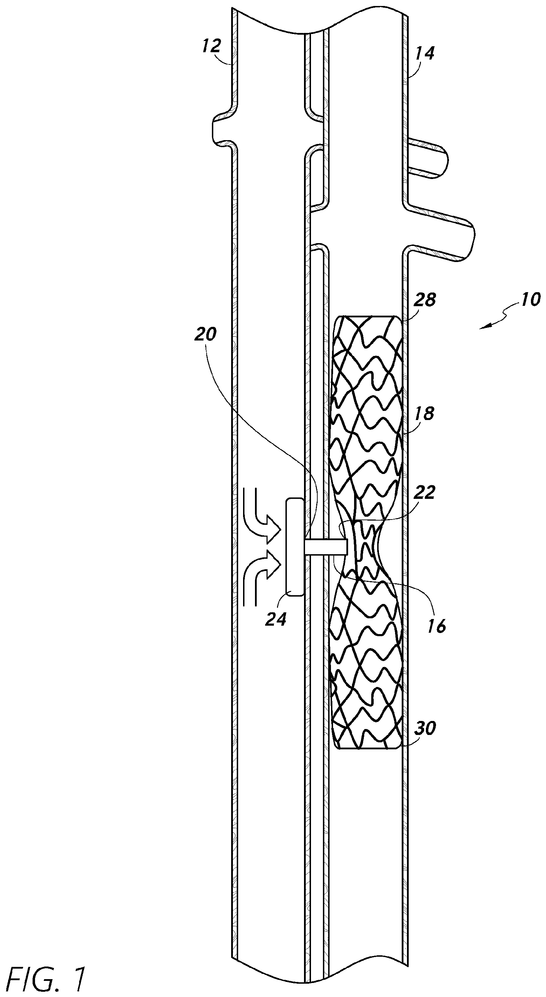

FIG. 1 illustrates a device 10 configured to be deployed or implanted in adjacent fluid vessels, such as a vein 12 (e.g., the inferior vena cava) and an artery 14 (e.g., abdominal aorta), according to one or more embodiments. For ease of illustration, FIG. 1 depicts the vein 12 and the artery 14 in a cross-sectional view and the device 10 in a non-cross-sectional view. The device 10 may include a shunt structure 16, 24 (also referred to as "the shunt portion 16, 24") configured to be implanted or deployed within anatomy between the vein 12 and artery 14 or at least partially within the vein 12 or the artery. When implanted or deployed, the shunt structure 16, 24 may extend between the vein 12 and the artery 14 or at least partially into the vein 12 or the artery 14. The device 10 may also include a conduit structure 18 (also referred to as "the stent portion 18") configured to be implanted or deployed within a fluid vessel, such as the artery 14 or the vein 12, in some instances. The conduit structure 18 may connect to the shunt structure 16, 24.

The shunt portion 16, 24 may include a lumen portion 16 or an anchor portion 24 (also referred to as "the anchor 24" or "the shunt anchor 24"). The lumen portion 16 may have an elongated form with a lumen therein. In some embodiments, the lumen portion 16 has a cylindrical, rectangular, or other form. The lumen portion 16 may have a shunt inlet end 20 configured to be positioned or implanted in the vein 12, and a shunt outlet end 22 configured to be positioned or implanted within the artery 14, thus providing a fluid passage between those blood vessels. The shunt inlet end 20 may be secured to the anchor portion 24, which can be a stent or similar structure adapted to be secured within the vein 12. The anchor portion 24 may help to hold the shunt inlet end 20 at a desired position, e.g., to prevent the shunt inlet end 20 from sliding in or out of the vein 12. In some embodiments, the anchor portion 24 may have a disk-like or tapered form and may be referred to as "the disk-like or tapered structure 24." In some embodiments, the anchor portion 24 may be implemented as a stent adapted to be deployed within the vein 12, in a similar fashion as the stent portion 18 depicted as being deployed within the artery 14. The shunt outlet end 22 may be secured to the stent portion 18, which is deployed within the artery 14.

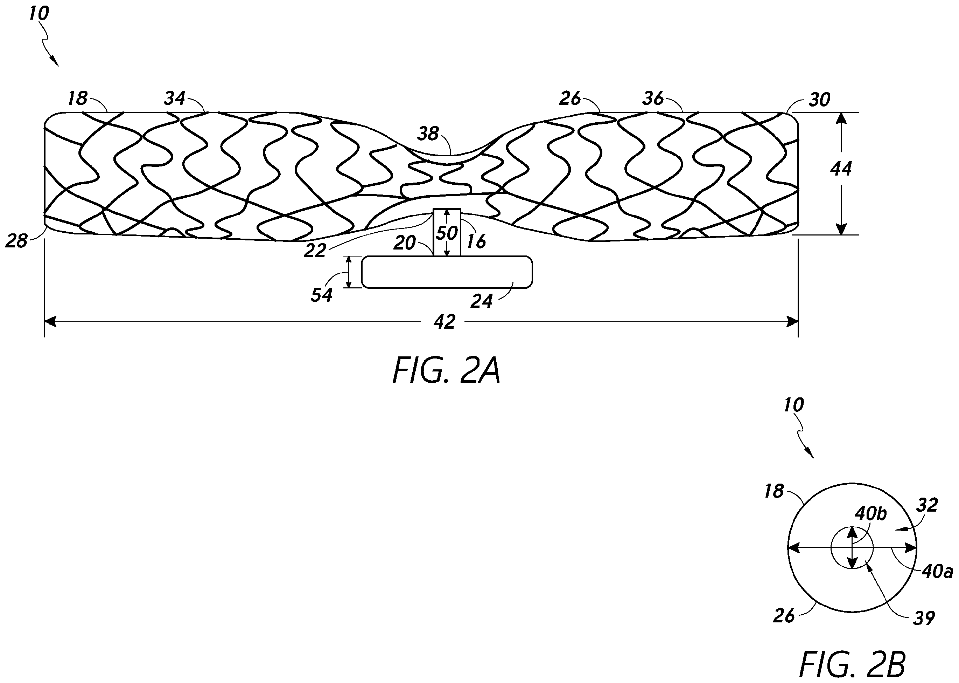

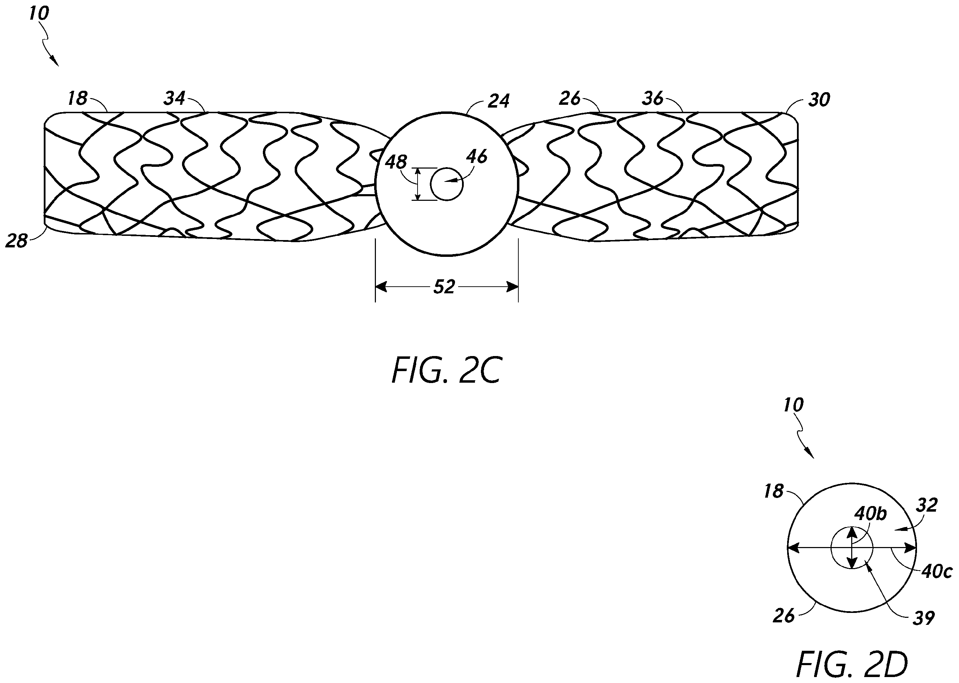

The device 10 is depicted in greater detail in FIGS. 2A-2D. The stent portion 18 has stent wall 26 extending between an inlet end 28 and an outlet end 30, with a stent lumen 32 passing within the stent wall 26 between the inlet and outlet ends 28, 30. The stent inlet portion 34 and stent outlet portion 36 may be separated by a tapered mid-portion 38 (also referred to as "the narrowed portion 38" or "the constricted portion 38") where the stent lumen 32 narrows. The tapered mid-portion 38 defines an orifice 39 having a narrower internal diameter 40b than the inlet diameter 40a. It may also be narrower than the outlet diameter 40c. The inlet diameter 40a may depend on the particular application, such as the characteristics of the blood vessel in which the stent portion is deployed. For example, for a stent portion adapted for deployment in the intrarenal abdominal aorta, the inlet diameter 40a may be from 1.5 to 4 cm. The orifice internal diameter 40b may be 0.90 or less of the inlet diameter 40a; 0.80 or less of the inlet diameter 40a; 0.70 or less of the inlet diameter 40a; 0.60 or less of the inlet diameter 40a; 0.50 or less of the inlet diameter 40a; 0.40 or less of the inlet diameter 40a; or smaller. Other dimensions are also within the scope of the disclosure.

The stent portion 18 may be dimensioned to be deployed within a desired fluid vessel, such as the abdominal aorta. The stent portion 18 has a stent length 42 and a stent outer diameter 44. The stent outer diameter 44, when in the deployed configuration, may be adapted to match the internal diameter of a desired blood vessel so that the stent wall 26 engages the walls of the blood vessel. The stent length 42 and diameter 44 may depend on the particular application, including the vessel in which the stent portion is to be deployed. For example, in an abdominal aortic application, the stent length 42 may be from 40 to 80 mm, while the diameter 44 may be from 1.5 to 4 cm. Other dimensions are also within the scope of the disclosure.

The lumen portion 16 may have a shunt lumen 46 extending between the shunt inlet end 20 and shunt outlet end 22. The shunt lumen diameter 48 may be constant along the shunt length 50, or may vary along the length 50 such as via tapering, etc. In some embodiments, the anchor portion 24 may be dimensioned to firmly seat the anchor portion 24 within the vein 12 but without substantially blocking blood flow through the vein 12. For example, the anchor portion 24 may have a diameter 52 sufficient to seat the anchor portion 24 firmly in a desired position in the vein 12 (e.g., the diameter 52 may be within a particular range, above or below a threshold, etc.), or may have a thickness 54 small enough (e.g., within a particular range, below or above a threshold, etc.) so that the anchor portion 24 does not extend too far into the vein 12 (e.g., does not extend more than a particular distance), thus preventing the anchor portion 24 from substantially blocking blood flow within the vein 12. The shunt length 50 may be sufficient to cross the distance between the adjacent blood vessels in which the device 10 is deployed, with the shunt lumen diameter 48 selected to accommodate sufficient blood flow therethrough as desired for a particular application.

In some embodiments, any of the elements of the device 10, such as the conduit structure 18, the lumen portion 16, or the anchor portion 24, can be formed of a flexible material or mesh, or a wire-frame structure, such as a stent or stent-like structure. In examples, a flexible material or mesh, or a wire-frame structure may maintain a particular form unless sufficient force is applied. In some embodiments, a flexible material or mesh or a wire-frame structure may be formed of metal, plastic, silicone, or another material. Further, in some embodiments, a flexible material or mesh or a wire-frame structure may include a covering (also referred to as a "lining"), which may be disposed on the outside or interior portion of a frame structure. Although the conduit structure 18 is illustrated in many figures as being implemented as a stent, the conduit structure 18 may take other forms. For example, the conduit structure 18 may be implemented as a substantially cylindrical or rectangular structure or tube formed of metal, plastic, or another substance.

Although FIG. 1 and other figures depict the walls of the artery 14 along the stent portion 18 as having a substantially uniform diameter, the walls may be formed to a narrowed region of the stent portion 18, in some cases. For example, in some embodiments, the walls of the artery 14 near the narrowed region of stent portion 18 (e.g., where the lumen portion 16 connects to the stent portion 18) may deform or collapse inward toward the stent portion 18 after deployment of the device 10 or over time to conform to the outer walls of narrowed region of the stent portion 18. In other embodiments, the walls of the artery 14 near the narrowed region may remain with a relatively uniform diameter, as shown in FIG. 1.

In some embodiments, the disclosure describes systems, devices, and methods that are based on the Venturi effect, where a reduction in fluid pressure occurs when fluid flows through a constricted area of a lumen. Using Bernoulli's equation in the case of incompressible, inviscid flows (such as the flow of water and other liquids), a theoretical pressure drop at such a constriction may be given by:

$$p_1 - p_2 = \frac{\rho}{2}\left(v_2^2 - v_1^2\right) \quad Q = v_1 A_1 = v_2 A_2$$

where v = fluid velocity at points 1 and 2; $A$ = cross-sectional area at points 1 and 2; and $\rho$ = density.

In some embodiments of the current disclosure, the corresponding Bernoulli equation can be developed by the following:

$$P_1 - P_2 = \frac{\rho}{2}\left(v_2^2 - v_1^2\right)$$

$$v_1 A_1 = v_2 A_2$$

$$v_2 = v_1 \frac{A_1}{A_2} = v_1 \left(\frac{D_1}{D_2}\right)^2$$

$$P_1 - P_2 = \frac{\rho}{2}\left(v_1^2 \left(\frac{D_1}{D_2}\right)^4 - v_1^2\right)$$

$$P_2 = P_1 - \frac{\rho v_1^2}{2}\left(\left(\frac{D_1}{D_2}\right)^4 - 1\right)$$

In some embodiments, the pressure at the orifice to draw blood through the shunt can thus be calculated as:

$$P_2 = P_1 - 0.00106 \left[\frac{kg}{cm^3}\right]\left\{v_1^2 \left[\left(\frac{A_1}{A_2}\right)^2 - 1\right]\right\}$$

where $\rho = 0.00106 \left[\frac{kg}{cm^3}\right]$ for blood.

Using known values (means or averages) of abdominal aortic pressure, velocity, and diameter, changes in blood pressure $P_2$ within an orifice can be calculated for various parameters:

| P$_1$ | V$_1$ | D$_1$ | D$_2$ (neo-orifice) | P$_2$ |
|---|---|---|---|---|
| Abdominal Aorta Pressure | Abdominal Aorta Velocity | Abdominal Aorta Diameter | Device Orifice | Orifice Pressure |
| [mm Hg] | [cm/sec] | [cm] | [cm] | [mm Hg] |
| 90 | 20 | 2 | 0.4 | -9.2 |
| 80 | 20 | 1.8 | 0.35 | -31.1 |
| 80 | 30 | 2 | 0.5 | -11.2 |
| 90 | 30 | 2.5 | 0.6 | -17.5 |

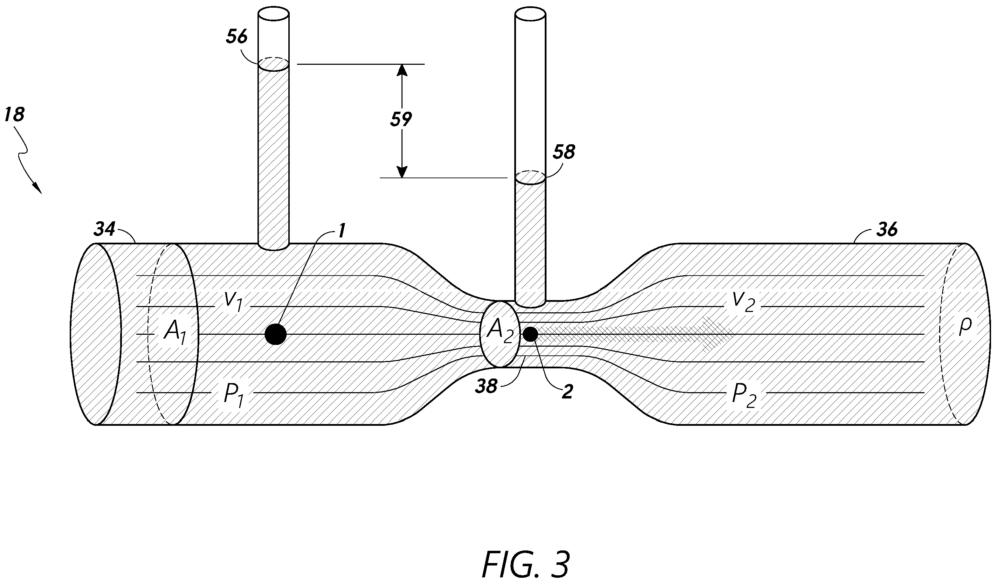

Example principles of operation of one or more devices of the disclosure are shown in FIG. 3. Blood flow passing from the inlet portion 34 to the outlet portion 36 passes through the narrowed tapered portion 38. Due to the increased speed of the blood flow through the narrow-tapered portion 38, the inlet portion blood pressure 56 and outlet portion blood pressure are higher than the narrowed portion blood pressure 58. The blood pressure drop 59 helps to pull blood from the vein through the shunt and into the stent portion in the artery, which can increase arterial pressure while reducing vein pressure.

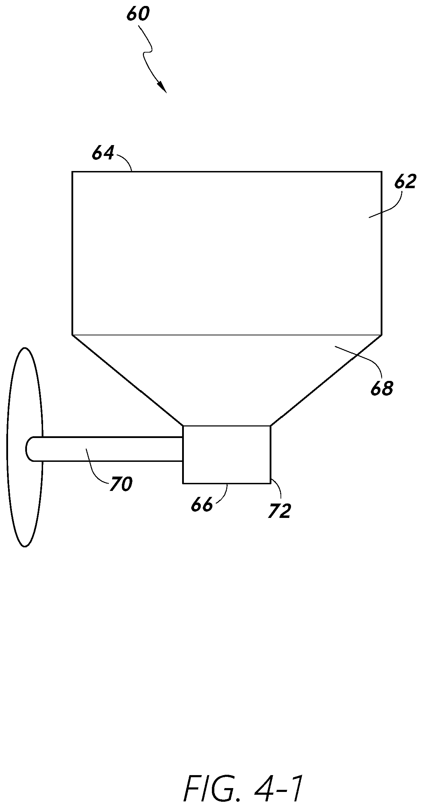

In some embodiments, stents according to the disclosure may be of an hourglass shape such as in FIGS. 1 and 2A-2D, or may be in other shapes. For example, as depicted in FIG. 4-1, a device 60 may have a stent portion 62 having a single funnel shaped portion 68, with a relatively wide inlet end 64 and a narrower outlet end 66. The outlet end 66 may be at a distal end of the tapered or funnel portion 68, with the shunt portion 70 (also referred to as "the lumen portion 70") ending within the narrow orifice portion 72 at the downstream end of the tapered or funnel portion 68.

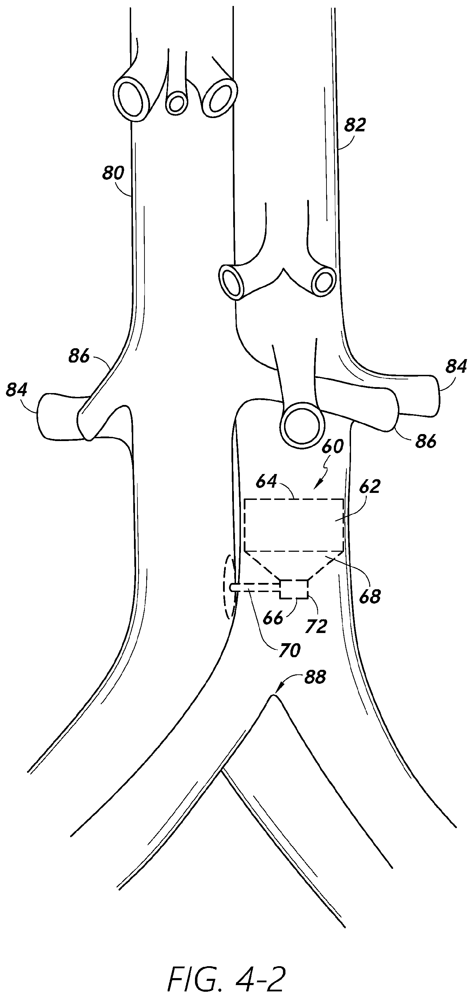

As shown in FIG. 4-2, the device 60 (and other devices discussed herein) may be positioned in or between the inferior vena cava 80 and abdominal aorta 82 of a patient. In some embodiments, the device 60 may be positioned distally of the renal arteries 84 and renal veins 86, or may be positioned proximal of the aortic bifurcation 88. However, the device 60 and any other device discussed herein may be positioned at other locations, such as other locations within the aorta, vena cava, or other anatomy. In some embodiments, a device is positioned within or between other fluid vessels, such as other blood vessels which may be located adjacent to each other or separated by a distance.

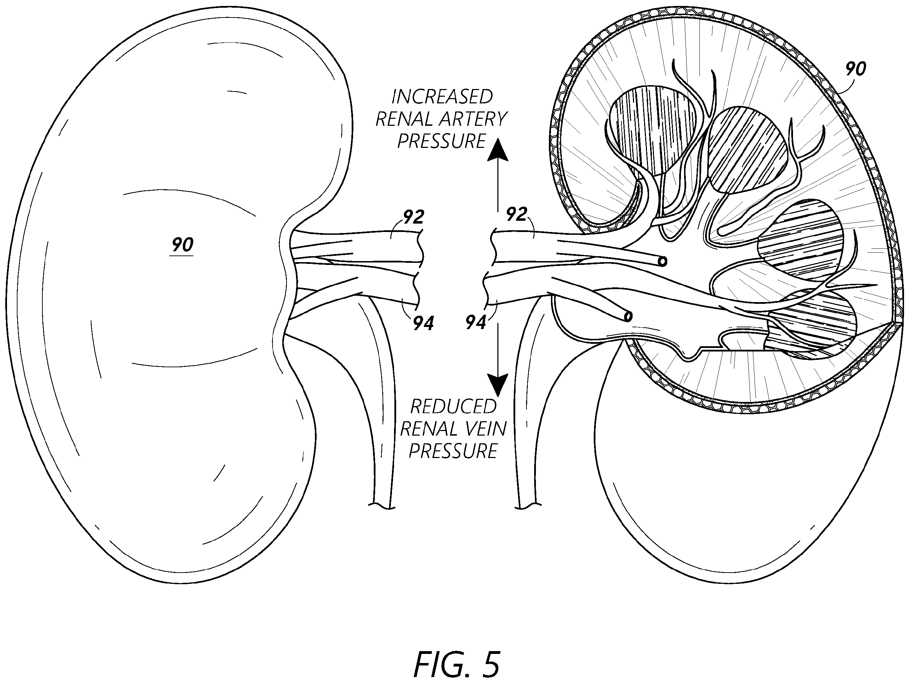

When deployed to pull blood from the inferior vena cava into the abdominal aorta, such as depicted in FIGS. 1 and 4, a device of the disclosure can improve function of the kidneys 90 as shown in FIG. 5. For example, the device can increase the blood pressure within the abdominal aorta, which can lead to increased blood pressure within the renal arteries 92. Such deployment of the device may also reduce the blood pressure within the inferior vena cava, which can lead to reduced blood pressure within the renal veins 94. The combination of increased renal artery pressure with reduced venal vein pressure can increase the flow of blood through the kidneys 90, which can improve renal function.

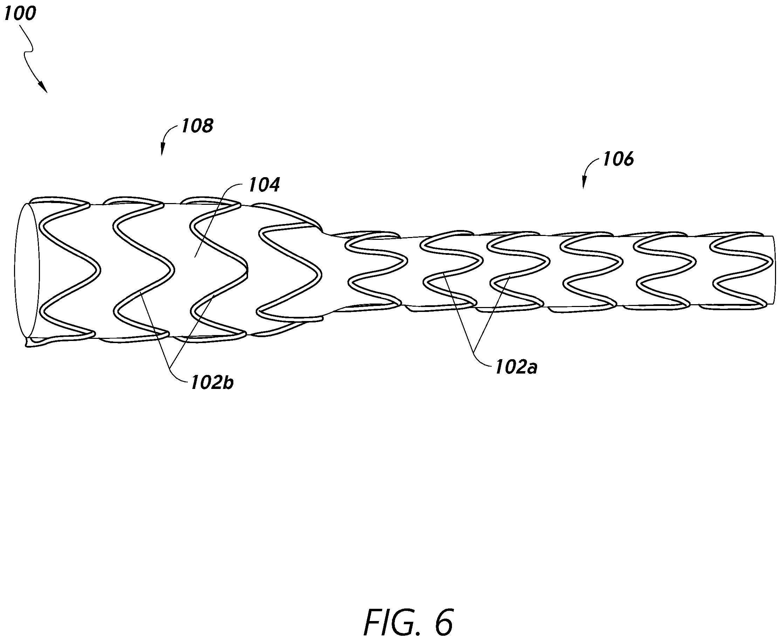

Stent portions may be in various forms. For example, a graft-like stent portion 100 may be formed from multiple stent-like portions 102a, 102b secured to a liner 104 in a graft-like configuration, as shown in FIG. 6. The stent-like portions 102a within a narrowed portion 106 of the stent portion 100 may be dimensioned to have a smaller diameter than that of the stent-like portions 102b within a wider portion 108 of the stent portion 100. The individual stent-like portions 102a, 102b may be independently expandable from each other, such as via being released individually from a restraining catheter sheath or individually expanded via catheter balloon expansion. The graft-like stent portion 100 may be in a funnel shape (similar to that depicted in FIGS. 4-1, 4-2, and 6), an hourglass shape (similar to that depicted in FIG. 1), or in other forms.

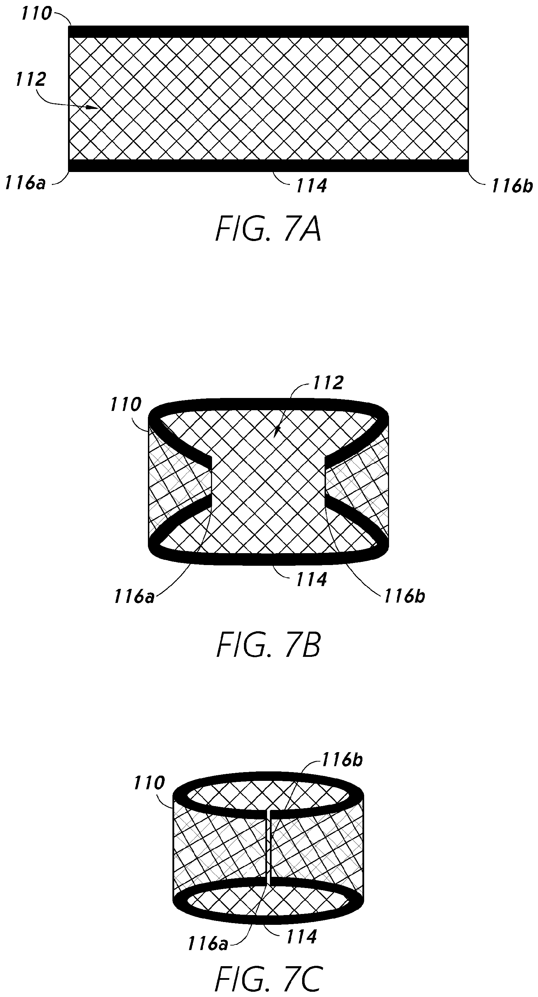

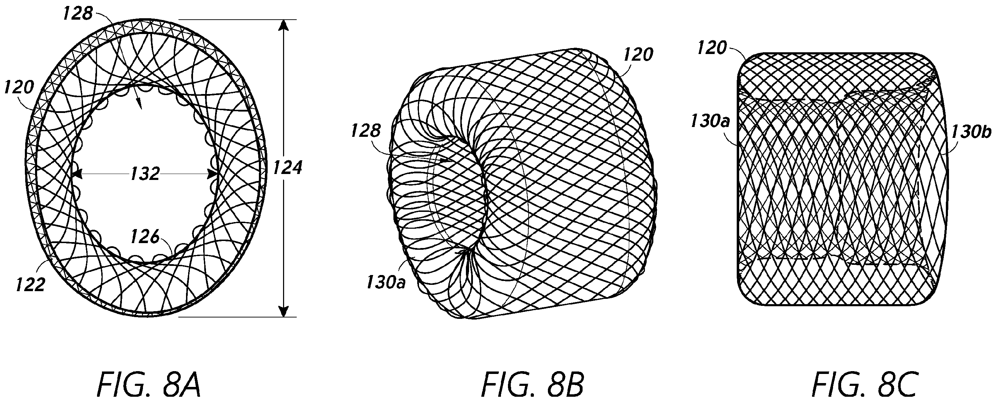

In some embodiments, a stent portion can include an elongate torus-like form. For example, an area of suddenly narrowed diameter can be provided in a device without the relatively large tapering sections on either side of the orifice as depicted in FIGS. 1 and 2A-2D. To illustrate, an area of narrowed diameter lumen can extend the entire length of the device or substantially the entire length of the device, such as is depicted in the cross-sectional side views in FIGS. 7A-7C. In some embodiments, to create a stent portion, a metal-mesh tube 110 having a tube lumen 112 and a tube wall 114 having opposing ends 116a, 116b (FIG. 7A) may be provided. The opposing ends 116a, 116b can be inverted into the tube lumen 112 (FIG. 7B) until the opposing ends 116a, 116b meet each other (FIG. 7C). The opposing ends 116a, 116b may be secured to each other, such as via suture, wire connections, or otherwise. The resulting stent portion 120 (FIGS. 8A-8C) may have an outer wall portion 122 defining the stent outer diameter 124, and an inner wall portion 126 defining the stent inner lumen 128. Opposing inlet and outlet ends 130a, 130b are on either end of the stent inner lumen 128. The stent inner lumen 128 may be narrower in diameter 132 than the outer wall portion 122, thus defining a stent inner lumen 128 which is narrower (e.g., substantially narrower) than the blood vessel lumen in which the device is implanted. In some embodiments, the stent portion 120 may have an open mesh, but with sufficiently thick mesh weave (e.g., satisfying a weave parameter, range, threshold, etc.) to cause much of the blood flow to be diverted into the orifice or inner lumen 128, resulting in increased blood flow speed and reduced blood pressure within the orifice or inner lumen 128. Further, in some embodiments, the stent portion 120 may be combined with a lining covering the inner wall portion 126 to divert blood flow (e.g., substantially all, a particular amount, etc.) through the orifice or inner lumen 128, resulting in increased blood flow speed and reduced blood pressure within the orifice or inner lumen 128. Such stent portion 120 can be combined with a shunt portion and other elements, such as those depicted elsewhere in this application, with an inlet end of the shunt secured to the inner wall portion 126 to provide a fluid connection through the shunt and into the orifice or inner lumen 128 to form a device.

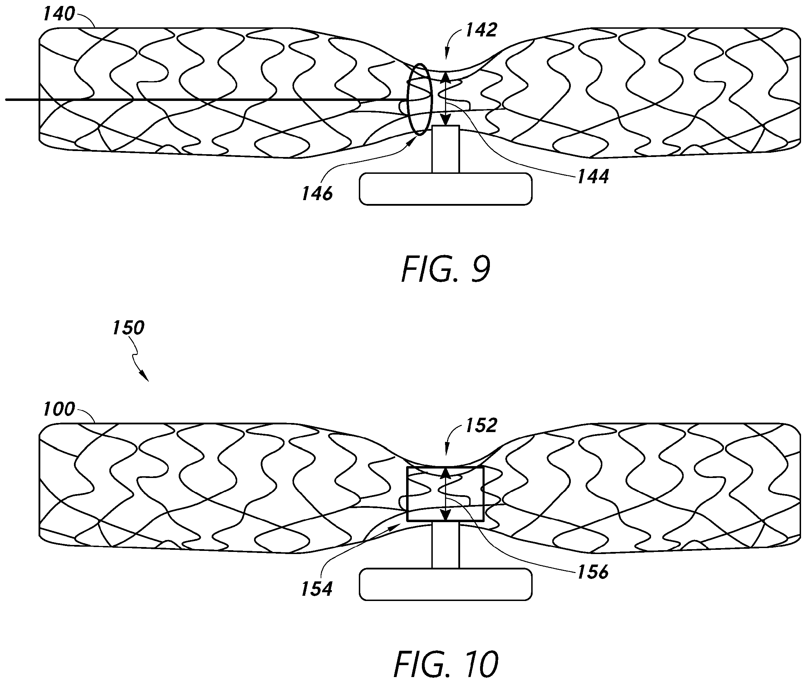

In some embodiments, a stent portion 140 may have an orifice 142 with an adjustable orifice diameter 144, as shown in FIG. 9. The orifice diameter 144 may be adjusted via a lasso suture 146 or another mechanism which can tighten and loosen the orifice 142 to vary the orifice diameter 144. Such adjustments may be particularly applicable for stent portions 140 having frames formed from self-expanding material, such as Nitinol, etc. The orifice diameter adjustment may be made by a surgeon or other user, who may review the patient's hemodynamics response (e.g., using echo flow assessment, etc.) before, during, or after device deployment to assess the desired modification to be provided to the orifice diameter 144 using the lasso suture 146 or other mechanism. Such a lasso suture or mechanism can be combined with various deployment procedures, such as any of the deployment methods disclosed herein. In such procedures and methods, the adjustment of the orifice diameter 144 may occur prior to advancing the device within the patient, during initial deployment, immediately after initial deployment, or at a later time, such as after a delivery catheter has been fully removed from the patient. In some embodiments, the lasso may remain secured to the device for later adjustments of the orifice diameter 144, with a proximal end of the lasso positioned, e.g., just outside the patient's body, so that it can be accessed by a user.

FIG. 10 depicts an example stent portion 150 where a stent frame section 152 around an orifice 154 is expandable. For example, the stent frame section 152 may be formed from stainless steel, cobalt chromium, a plastically deformable material, or another substance. The orifice diameter 156 may be adjusted by a surgeon or other user by introducing a balloon catheter into the orifice 154 and expanding the balloon to enlarge the orifice diameter 156. Such orifice diameter adjustment may be made by a surgeon or other user during or after review of the patient's hemodynamics response (e.g., using echo flow assessment, etc.), which may occur before, during, or after device deployment to assess the desired modification to be provided to the orifice diameter 156 using a balloon or other expansion mechanism. Such expansion of the orifice diameter 156 may occur at the time that the device is initially deployed in the patient, or may be performed after device deployment.

In some embodiments, it may be desirable to expand an orifice of a stent portion after initial deployment. For example, in cases where a stent frame or other structure around the orifice is formed from a deformable material, a surgeon or other user can use a balloon catheter or other expansion mechanism to expand the orifice to a wider diameter, including a much wider diameter, such as where the orifice is expanded to a fully open position, e.g., until it is as wide as the blood vessel prior to treatment, etc. In cases where the stent frame or other structure around the orifice is formed from a self-expanding material, a surgeon or other user can loosen or cut the suture or other expansion control mechanism to quickly expand the orifice to the desired wider diameter. Such orifice widening may be provided in lieu of, or prior to, removal of the device from the patient. In some embodiments, removing the device from the patient may include drawing the device into a removal catheter, or back into the delivery catheter, such as via use of sutures or other mechanisms which can engage the device and pull it back into the catheter.

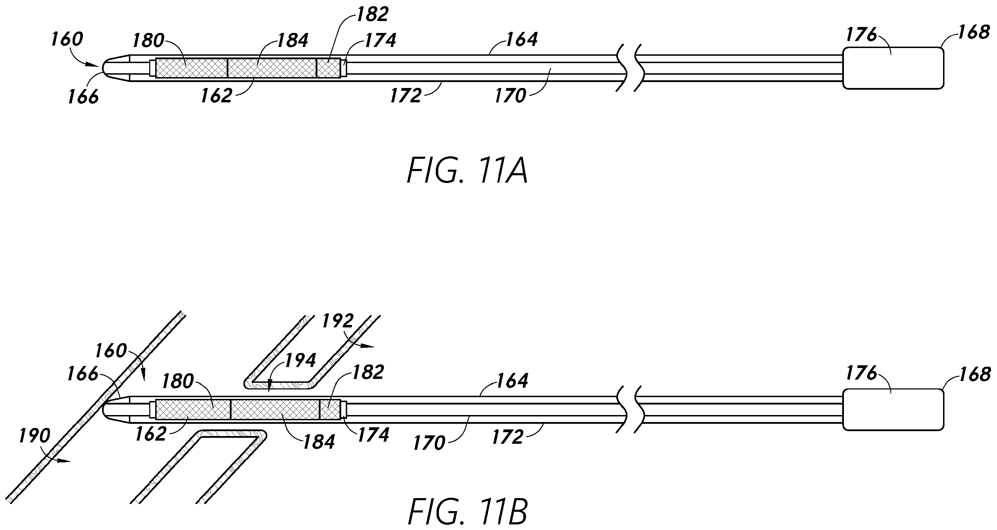

FIGS. 11A-11D illustrate an example system 160 that includes a device 162 and a delivery catheter 164 according to one or more embodiments. The delivery catheter 164 has a distal end 166 and proximal end 168, with the device 162 secured to the catheter 164 adjacent the distal end 166. The catheter 164 has an elongated main body 170, and may include a retractable sheath 172 which surrounds and protects the device 162 or portions thereof, and which may also prevent the device 162 or portions thereof from undesired expansion (such as where the device 162 or portions thereof are self-expanding). The catheter 164 may include a balloon 174 or other expansion mechanism which can mechanically expand portions of the device 162.

In some embodiments, a handle 176 may be provided at the proximal end 168 of the catheter 164. The handle 170 may include controls, such as controls for retracting the sheath or for expanding or contracting the balloon or other expansion mechanism.

The device 162 may include a first portion 180 for deployment in a first blood vessel, a second portion 182 for deployment in a second blood vessel, or a lumen portion 184 connecting the first and second portions 180, 182 and adapted to extend between the first blood vessel and the second blood vessel. In some embodiments, the second portion 182 or the lumen portion 184 may form a shunt structure or shunt portion. The device 162 may include any of the devices discussed herein.

In some embodiments, the system 160 can be advanced into a patient to deploy the device 162 in a first blood vessel 190 and second blood vessel 192. As depicted in FIG. 11B, the catheter 164 may be advanced via the second blood vessel 192 into the first blood vessel 190, which can be achieved with the catheter 164 passing through a passage 194 created by a surgeon, interventionist, or other user between the first blood vessel 190 and the second blood vessel 192. The catheter 164 may be advanced such that the device 162 is positioned with the first portion 180 within the first blood vessel 190 (such as an abdominal aorta), with the second portion 182 within the second blood vessel 192 (such as an inferior vena cava), or with the lumen portion 184 positioned within the passage 194.

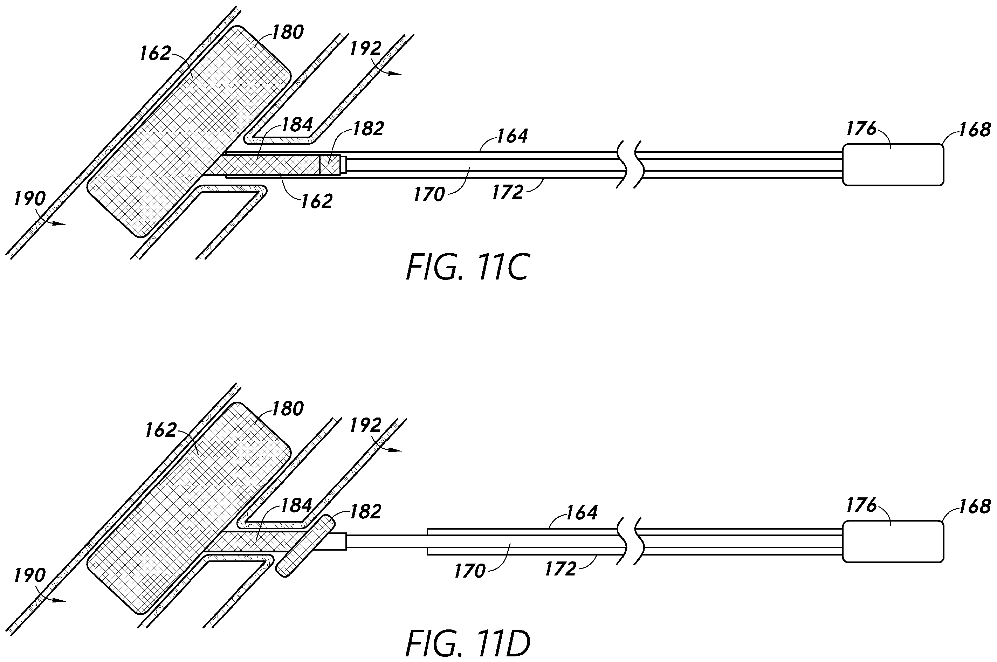

As shown in FIG. 11C, the first portion 180 can be expanded in position within the first blood vessel 190 (e.g., by retraction of the sheath 172, loosening of a lasso, or activation of an expansion mechanism, such as the balloon 174). The second portion 182 can then be released to expand or otherwise deployed within (e.g., adjacent a wall of) the second blood vessel 192 (e.g., by retraction of the sheath 172, loosening of a lasso, or activation of an expansion mechanism, such as the balloon 174), as depicted in FIG. 11D. In some embodiments, the shunt 184 may also be transitioned to a deployed configuration, such as via radially expansion (e.g., by retraction of the sheath 172, loosening of a lasso, or activation of an expansion mechanism, such as the balloon 174), which may occur after deployment or expansion of the first portion 180 or before deployment or expansion of the second portion 182. With the device 162 properly deployed, the catheter 164 can be removed from the patient. Although the first portion 180 is illustrated with a substantially uniform diameter in FIGS. 11C and 11D, the first portion 180 may have a narrowed region (e.g., where the lumen portion 184 connects to the first portion 180), as similarly discussed in other portions of the disclosure.

After the device 162 is deployed at a desired position, adjustments may be made to the orifice diameter, such as discussed and depicted with respect to FIGS. 9 and 10. In some embodiments, such adjustments may be made using the catheter 164 (e.g., using the catheter balloon 174). In some examples, the catheter 164 may not be removed from the patient until after the desired adjustments are made to the orifice diameter. In some examples, adjustments may be made in conjunction with review by the surgeon or other user of the patient's hemodynamics response (e.g., using echo flow assessment, etc.).

Although examples are discussed in the context of providing blood from the inferior vena cava into the abdominal aorta, any of the devices and methods discussed herein can be used to provide blood or another fluid from the abdominal aorta into the inferior vena cava or between other fluid vessels. For example, in some embodiments of the context of FIGS. 11A-11C, the first portion 180 of the device 162 may be deployed or positioned in the inferior vena cava and the second portion 182 of the device 162 may be deployed or positioned in the abdominal aorta. Here, blood from the abdominal aorta may pass into the inferior vena cava through the device 162.

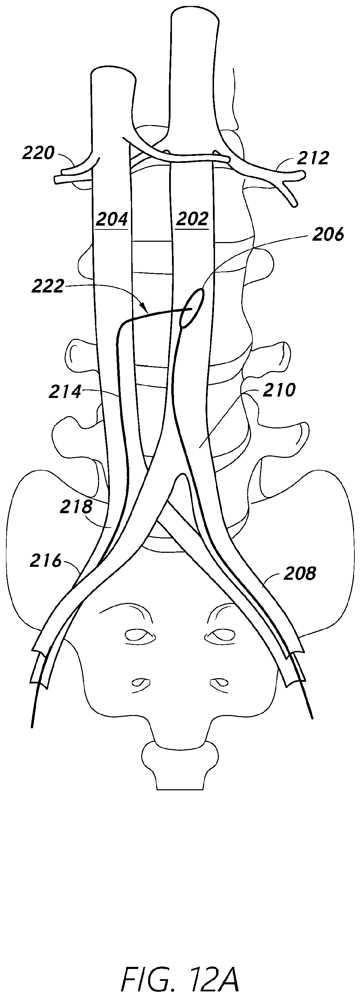

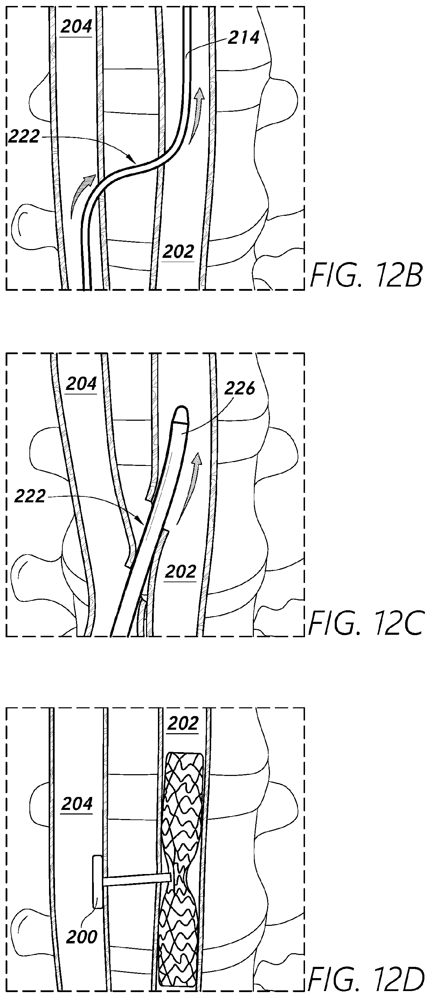

FIGS. 12A-12D depict an example method of deploying a device 200 to provide blood flow into a first blood vessel, such as the abdominal aorta 202, from a second blood vessel, such as the inferior vena cava 204, said method not being protected by the appended set of claims. A guidewire may be advanced between the inferior vena cava 204 and the abdominal aorta 202 (e.g., using snaring techniques, in some cases). For example, as depicted in FIG. 12A, a snare 206 may be advanced into the abdominal aorta 202 via a femoral artery 208 (such as via an incision in the patient which leads to the femoral artery 208) to a position between the arterial femoral bifurcation 210 and the renal arteries 212. A guidewire 214 may also be separately advanced into the inferior vena cava 204 via a femoral vein 216 (such as via an incision in the patient which leads to the femoral vein 216) to a position between the venal femoral bifurcation 218 and the renal veins 220. In some embodiments, advancement of the guidewire 214 may include or be preceded by advancement of a catheter or other element adapted to create the passage 222 (e.g., via puncture) between the inferior vena cava 204 and the abdominal aorta 202. The guidewire 214 may be advanced through or create the passage 222 into the abdominal aorta 202 and engage with the snare 206. In some embodiments, the snare 206 may be used as a landmark or target to assist in guiding the guidewire 214 into the abdominal aorta 202 (e.g., used with imaging to identify a target location to cross over from the inferior vena cava 204 into the abdominal aorta 202). Additionally, or alternatively, the snare 206 may be used to pull the guidewire 214 into the abdominal aorta 202. In some embodiments, once within the abdominal aorta 202, the guidewire 214 may be advanced further up the abdominal aorta 202 (i.e., toward the patient's heart), as depicted in FIG. 12B. While in other embodiments, the guidewire 214 may remain within proximity to an initial entry point into the abdominal aorta 202.

A delivery catheter 226 may be advanced via the femoral vein 216 (including via the puncture in the patient leading to the femoral vein 216) and into the inferior vena cava 204, through the passage 222, and into the abdominal aorta 202, as depicted in FIG. 12C. Advancement of the delivery catheter 226 may include advancing the delivery catheter along or over the guidewire 214. The delivery catheter 226 may be similar to that depicted in FIGS. 11A-11D.

The device 200 may then be deployed via the delivery catheter 226 at the desired location (e.g., using the catheter sheath withdrawal, balloon expansion, etc., such as via use of the devices and methods depicted and described with respect to FIGS. 11A-11D). After the device 200 is properly deployed, the delivery catheter 226 or guidewire 214 may be withdrawn from the patient. The device 200 after catheter withdrawal is depicted in FIG. 12D.

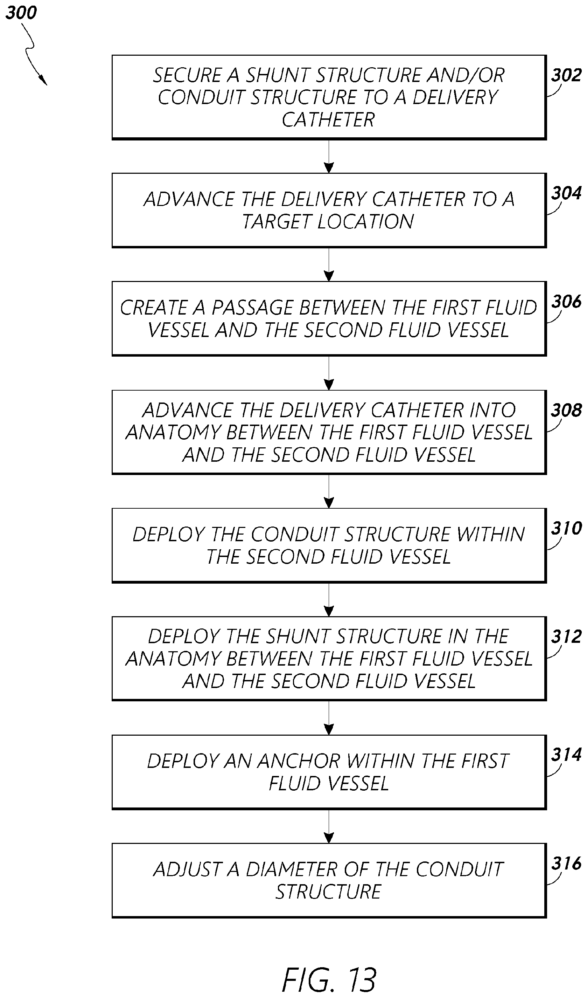

FIG. 13 illustrates an example process 300 for implanting a device within one or more vessels of a patient in accordance with one or more embodiments of the present disclosure. In some embodiments, the process 300 can be performed to implant the device within a first fluid vessel, such as an inferior vena cava, or a second fluid vessel, such as an aorta. For ease of discussion, many embodiments discussed below may refer to the aorta and the inferior vena cava and implanting particular structures within the aorta and the inferior vena cava. It should be appreciated that the process 300 may be used to implant the structures within other fluid vessels and the structures may be implanted differently within the aorta and the inferior vena cava than that described below.

At block 302, a shunt structure or a conduit structure may be secured to a delivery catheter. For example, the shunt structure or the conduit structure may be attached to the delivery catheter in a collapsed or other state for delivery of the structure within human anatomy. The shunt structure or the conduit structure may be attached to the delivery catheter prior to advancing the delivery catheter within the human anatomy.

At block 304, the delivery catheter may be advanced to a target location. For example, the delivery catheter may be advanced through a femoral vein and within the inferior vena cava to a desired location within the inferior vena cava where the delivery catheter is to cross over into the abdominal aorta (e.g., through a passage, as discussed below). In some embodiments, the delivery catheter is advanced over or along a guidewire that is located within the human anatomy.

At block 306, a passage may be created between the first fluid vessel and the second fluid vessel. In some embodiments, a guidewire is used to create the passage between the first fluid vessel and the second fluid vessel, such as before the delivery catheter is advanced to the target location. As such, in some cases, the block 306 may be performed before the block 304. Alternatively, or additionally, in some embodiments, the delivery catheter is used to create the passage. The first fluid vessel may be adjacent to the second fluid vessel, such as directly adjacent, within a particular distance, etc.

At block 308, the delivery catheter may be advanced into anatomy between the first fluid vessel and the second fluid vessel. For example, the delivery catheter may be advanced from the inferior vena cava, through the passage, and into the abdominal aorta.

At block 310, the conduit structure may be deployed within the second fluid vessel. For example, the conduit structure may be implanted within the abdominal aorta. **In** some embodiments, the delivery catheter includes a sheath. The conduit structure may be deployed by slidingly retracting the sheath with respect to the conduit structure or radially expanding the conduit structure. In some embodiments, the conduit structure includes a first section, a second section, and a lumen extending through the first section and the second section. In some examples, a diameter of the second section may be smaller than a diameter of the first section. Further, in some examples, the conduit structure may be self-expanding.

At block 312, the shunt structure may be deployed in the anatomy between the first fluid vessel and the second fluid vessel. For example, the shunt structure may be implanted within the passage or at least partly within the inferior vena cava with the shunt structure being connected to the conduit structure. In some embodiments, the delivery catheter includes a sheath. The shunt structure may be deployed by slidingly retracting the sheath with respect to the shunt structure or by radially expanding the shunt structure. In some embodiments, the shunt structure is self-expanding. Further, in some embodiments, the shunt structure is deployed after the conduit structure is deployed.

At block 314, an anchor may be deployed within the first fluid vessel. For example, the shunt structure may include the anchor, which may be deployed in the inferior vena cava. In some embodiments, the anchor is deployed after the shunt structure is deployed. Further, in some embodiments, deploying the shunt structure or deploying the anchor may seal (or include sealing) the passage such that fluid flow through the passage is prevented except for fluid flow through the shunt structure.

At block 316, a diameter of the conduit structure may be adjusted. For example, the second section of the conduit structure (e.g., a narrowed portion) or another section of the conduit structure may be reduced or increased in diameter. In some embodiments, a tensioning line may be used to reduce the diameter of the second section of the conduit structure. The tensioning line may extend through the conduit structure. Alternatively, or additionally, in some embodiments, a balloon on the delivery catheter may be used to increase or reduce the diameter of the second section of the conduit structure. The diameter of the conduit structure may be adjusted upon deploying the conduit structure within the second fluid vessel.

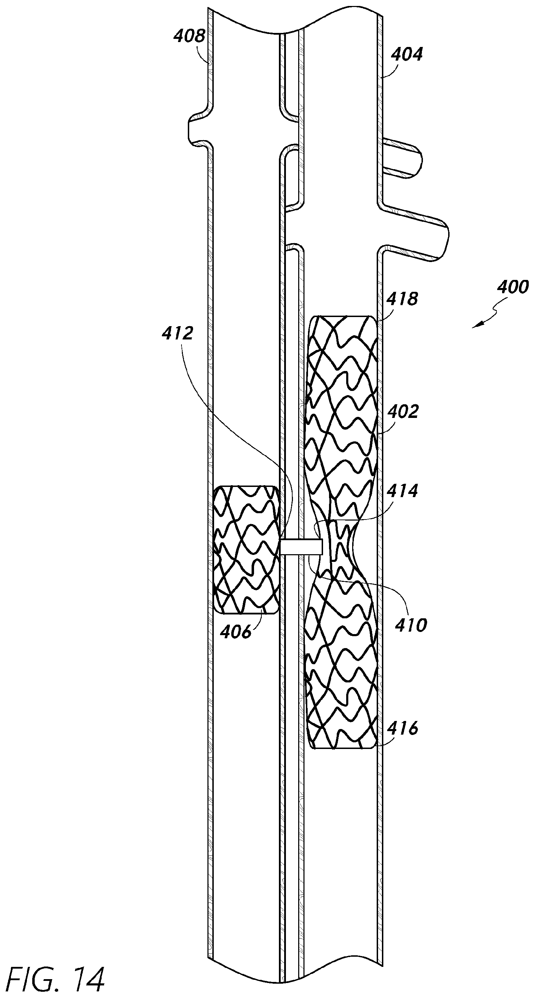

FIG. 14 illustrates an example device 400 with an anchor implemented as a stent according to one or more embodiments. The device 400 may include a conduit structure 402 configured to be implanted within a first fluid vessel, such as an aorta 404, or a shunt structure 406, 410 configured to be implanted within a second fluid vessel, such as an vein 408, or within anatomy between the first fluid vessel and the second fluid vessel. In examples, the device 400 may be configured to draw fluid, such as blood, from the vein 408 into the aorta 404 through the shunt structure 406, 410.

The conduit structure 402 may have an inlet end 418 (e.g., first end) and an outlet end 416 (e.g., second end), with a stent lumen passing within. The conduit structure 402 may have a narrowed portion or section, which is illustrated in the middle of the conduit structure 402 in FIG. 14. It should be appreciated that the narrowed portion or section may be located at other locations along the conduit structure 402. The narrowed portion or section may have a smaller diameter than the inlet end 418 or the outlet end 416.

The shunt structure 406, 410 may include a lumen portion 410 or an anchor portion 406. The lumen portion 410 may have an elongated form with a lumen therein. The lumen portion 410 may have a shunt inlet end 412 (e.g., first end) configured to be positioned or implanted in the vein 408, and a shunt outlet end 414 (e.g., second end) configured to be positioned or implanted within the artery 404 or connected to the narrowed section of the conduit structure 402. The anchor portion 406 may be configured to hold the lumen portion 410 in the appropriate position, such as the position illustrated in FIG. 14. In this example, the anchor portion 406 is implemented as a stent that is configured to expand radially during implantation to contact one or more walls of the vein 408, as shown in FIG. 14. In examples, the stent may include similar materials and structure as the conduit structure 402 (when the conduit structure 402 is implemented as a stent), except without a narrowed section. For example, the anchor portion 406 may include a flexible material or mesh or a wire-frame structure. The anchor portion 406 may include a lumen to allow fluid to flow through the anchor portion 406 or into the lumen portion 410. In some embodiments, the anchor portion 406 may have a smaller length than the conduit structure 402, as shown in FIG. 14. While in other embodiments, the anchor portion 406 may have the same or larger length than the conduit structure 402.

A fluid vessel can include any anatomy capable of carrying fluid, such as arteries, veins, capillaries, etc. Although certain embodiments are disclosed herein with respect to blood vessels (e.g., vessels of the aortic or venous systems), it should be understood that any of the disclosed devices may be implanted at least partially within any vessel, organ, or chamber of a patient's anatomy, including any chamber or vessel associated with the heart or cardiac circulatory system. As used herein, the term "fluid" may refer to a gas, a liquid, or a combination of the two.

Stent frames for use in one or more of the devices discussed herein can be formed of various materials, depending on the particular application and structures desired. For example, Nitinol, CoCr, stainless steel, and other metals may be used, as can polymers or other materials. In some embodiments, the stent frames can be cut (e.g., laser cut) from tubes or flat forms, made from circles which are formed into expandable shapes (such as zig-zag shapes), formed from wire braids or other wire forms, including mesh-like structures, etc. Linings can be made from various materials, such as woven materials, thin mesh-like materials, etc. Example liner materials include fabrics, polymers, ePTFA, sutures, electrospun materials, and tissues (e.g., pericardium). Any of these frame or liner materials, and any combination of them, can be used in any of the devices depicted and described herein.

Various approaches for treatments, including advancing catheters into position via a sheaths, are within the scope of the disclosure. In some embodiments, a transcatheter approach is implemented via a femoral artery. For instance, an implantation procedure can be similar to a transcatheter aortic valve replacement (TAVR) or transcatheter aortic valve implantation (TAVI) procedure that uses transcaval access.

In some example procedures of deploying a device, femoral artery access is obtained via an access sheath which is dimensioned for use in TAVR procedures. An incision may be created in the patient, leading to an internal blood vessel, such as a femoral artery. The distal end of the access sheath may be advanced through the incision and femoral artery and into a desired position within the aorta, with a hub positioned just outside the patient adjacent the incision or access site. A guide wire may be advanced from the femoral access site (and thru the aortic arch and into the patient's left ventricle, in cases of deploying a prosthetic aortic valve, for example). The steerable shaft of the imaging catheter or delivery catheter can be advanced over the guide wire, such as via standard over-the-wire techniques, to advance the distal end of the device to the target location. For example, the device may have a guide wire lumen. Echo, fluoroscopic, or other visualization techniques may be used as well as the electrophysiological 3D mapping techniques. The treatment or implant deployment can occur, such as by deploying a device at the target location. Once the proper deployment is confirmed, the catheter can be removed from the patient, the guidewire can be removed from the patient, the sheath can be removed from the patient, and the incision(s) closed, such as via sutures.

As noted above, the present disclosure is directed to devices, systems, and methods for providing fluid connections between blood vessels, such as may be desired to treat congestive heart failure or other conditions

In some embodiments, a device has a first blood vessel portion, a second blood vessel portion, and a shunt portion connecting the first blood vessel and second blood vessel portions. The first blood vessel portion is adapted to be deployed in a first blood vessel, such as an artery, and is shaped so as to create a local low pressure in the first blood vessel to thus pull fluid from the second blood vessel (e.g., a vein), through the shunt, and into the first blood vessel. The result is a reduction in load on the second blood vessel system (e.g., the venous system). When implanted between an abdominal aorta and an inferior vena cava, the renal function may improve due to the elevated pressure in the abdominal aorta and renal arteries, as well as the reduced pressure in the inferior vena cava and renal veins.

In some embodiments, devices for providing blood flow between blood vessels of a patient may include a stent portion having a stent outer wall defining a stent lumen extending between a stent inlet and a stent outlet. The stent portion may have a stent constricted portion positioned between the inlet and the outlet. The stent inlet may have a stent inlet lumen diameter, the stent outlet may have a stent outlet lumen diameter, and the stent constricted portion may have a constricted lumen diameter. The constricted lumen diameter may be less than 0.80 of the stent inlet diameter or the stent outlet diameter. The stent portion may be adapted to be positioned in a first blood vessel with the stent wall radially engaging a blood vessel wall of the first blood vessel. A shunt device may include a shunt portion having a shunt inlet and a shunt outlet on opposing ends of the shunt portion, with a shunt lumen extending from the shunt inlet to the shunt outlet. The shunt outlet may be secured to the stent constricted portion with the shunt lumen in fluid communication with the stent lumen, with the shunt portion extending in a non-parallel direction from the stent portion such that the shunt inlet is positioned away from the stent portion. The shunt lumen diameter may be no greater than, or less than, the constricted lumen diameter. The shunt lumen diameter may be 0.80 or less of the constricted lumen diameter.

Shunt devices according to the disclosure may include a shunt anchor secured to the shunt inlet, with the shunt anchor adapted to be positioned against a wall of a second blood vessel such as inferior vena cava. The shunt anchor may be a secondary stent, with the secondary stent adapted to be radially deployed within the second blood vessel and into contact with a wall of the second blood vessel.

The stent portion may be self-expanding or may have a self-expanding stent frame such as a memory-metal frame, e.g., formed of Nitinol. The self-expanding stent frame may have a delivery configuration and an expanded configuration, and the self-expanding stent frame may be biased toward the expanded configuration. The stent portion and the shunt portion may both be self-expanding, may both be formed of memory material, or may be biased toward an expanded configuration.

The shunt device may be adapted for deployment in a bifurcated section of blood vessel. The stent portion may include a bifurcation between the constricted portion and the stent outlet, with the stent portion having a secondary stent outlet downstream of the bifurcation.

The stent outlet lumen diameter may be the same as the constricted lumen diameter or may be substantially larger than the constricted lumen diameter.

In some embodiments, systems for providing blood flow between blood vessels of a patient may include any of the shut devices discusses herein combined with a delivery catheter. The delivery catheter may include a distal portion, an elongated central portion, and a proximal portion. The distal portion may be adapted to hold the device in a compressed delivery configuration, with the distal portion and elongated central portion adapted to be percutaneously advanced into a patient. The delivery catheter distal portion may have a sheath adapted to slidingly receive the stent portion, shunt portion, or shunt anchor of the shunt device, such as where the shunt device or portions thereof are self-expanding. The delivery catheter distal portion may include a radially expandable balloon adapted to radially expand one or more portions of the shunt device.

The catheter distal portion may be adapted to be percutaneously advanced to the aorta via a femoral vein and an inferior vena cava and through a passage created between the inferior vena cava and the aorta.

In some embodiments, methods may include providing a fluid connection between blood vessels, which may include providing a system having a delivery catheter such as those disclosed herein and a shunt device such as those disclosed herein. The methods may include creating a connecting passage between a first blood vessel and a second blood vessel; advancing the catheter distal portion with the shunt device secured thereto to a desired treatment site, the advancing comprising advancing the catheter distal and with the shunt device secured thereto through the first blood vessel and through the connecting passage and into the second blood vessel; deploying the stent portion within the second blood vessel; and deploying the shunt portion within the connecting passage with the shunt outlet in fluid communication with the second blood vessel. Deploying the shunt portion within the connecting passage may occur after deploying the stent portion within the first blood vessel. Where the shunt device has an anchor device secured to the shunt outlet, methods may also include deploying the anchor device within the second blood vessel. Deploying the shunt portion within the connecting passage may occur after deploying the stent portion within the first blood vessel, and deploying the anchor device within the second blood vessel may occur after deploying the shunt portion within the connecting passage.

After deployment of the shunt portion within the passage or after deployment of the stent portion within the first blood vessel, the passage extending between the blood vessels may be sealed by the shunt device such that fluid flow through the passage is prevented except for fluid flow through the shunt lumen.

The first blood vessel may be an aorta, and the second blood vessel may be an inferior vena cava. Advancing the catheter distal portion with the shunt device secured thereto to a desired treatment site may include advancing the delivery catheter distal portion through a femoral vein and into the inferior vena cava.

In some embodiments, methods may include securing the shunt device to the catheter distal portion, which may occur during manufacturing of the system or may be performed by a surgeon, interventionalist, or other user just prior to use of the system in deploying the shunt device into the patient.

The system may have a tensioning line extending through the stent portion and adapted to adjust the constricted lumen diameter. In such a system, methods of deployment may include, after deploying the stent portion within the first blood vessel, applying tension to the tensioning line to reduce the constricted lumen diameter.

Where stent portions or shunt portions are expandable (e.g., self-expanding), deploying stent portions within blood vessels may include radially expanding the stent portion, and deploying the shunt portion within the connecting passage may include radially expanding the shunt portion.

For self-expanding shunt devices, a catheter distal portion may have a sheath. Deploying the stent portion within a blood vessel may include slidingly retracting the sheath portion with respect to the stent portion, and deploying the shunt portion within the connecting passage comprises may include retracting the sheath portion with respect to the shunt portion.

In some embodiments, any element of any embodiment and its respective elements disclosed herein can be used with any other embodiment and its respective elements disclosed herein.

All dimensions listed are by way of example, and devices according to the disclosure may have dimensions outside those specific values and ranges. The dimensions and shape of the device and its elements may depend on the particular application.

Unless otherwise noted, all technical and scientific terms used herein have the same meaning as commonly understood by one of ordinary skill in the art to which this disclosure belongs. In order to facilitate review of the various embodiments of the disclosure, the following explanation of terms is provided:

The singular terms "a", "an", and "the" include plural referents unless context clearly indicates otherwise. The term "or" refers to a single element of stated alternative elements or a combination of two or more elements, unless context clearly indicates otherwise.

The term "includes" means "comprises." For example, a device that includes or comprises A and B contains A and B, but may optionally contain C or other components other than A and B. Moreover, a device that includes or comprises A or B may contain A or B or A and B, and optionally one or more other components, such as C.

The term "subject" refers to both human and other animal subjects. In certain embodiments, the subject is a human or other mammal, such as a primate, cat, dog, cow, horse, rodent, sheep, goat, or pig. In one example, a subject is a human patient.

Although methods and materials similar or equivalent to those described herein can be used in the practice or testing of the present disclosure, suitable methods and materials are described below. In case of conflict, the present specification, including terms, will control. In addition, the materials, methods, and examples are illustrative only and not intended to be limiting.

The above description of embodiments of the disclosure is not intended to be exhaustive or to limit the disclosure to the precise form disclosed above. While specific embodiments, and examples, are described above for illustrative purposes, various equivalent modifications are possible within the scope of the disclosure, as those skilled in the relevant art will recognize. For example, while processes or blocks are presented in a given order, alternative embodiments may perform routines having steps, or employ systems having blocks, in a different order, and some processes or blocks may be deleted, moved, added, subdivided, combined, or modified. Each of these processes or blocks may be implemented in a variety of different ways. Also, while processes or blocks are at times shown as being performed in series, these processes or blocks may instead be performed in parallel or may be performed at different times.

Certain terms of location are used herein with respect to the various disclosed embodiments. Although certain spatially relative terms, such as "outer," "inner," "upper," "lower," "below," "above," "vertical," "horizontal," "top," "bottom," and similar terms are used herein to describe a spatial relationship of one device or element or anatomical structure relative to another device or element or anatomical structure, it is understood that these terms are used herein for ease of description to describe the positional relationship between elements or structures, as illustrated in the drawings. Spatially relative terms are intended to encompass different orientations of the elements or structures, in use or operation, in addition to the orientations depicted in the drawings. For example, an element or structure described as "above" another element or structure may represent a position that is below or beside such other element or structure with respect to alternate orientations of the subject patient or element or structure, and vice-versa.

Conditional language used herein, such as, among others, "can," "could," "might," "may," "e.g.," and the like, unless specifically stated otherwise, or otherwise understood within the context as used, is intended in its ordinary sense and is generally intended to convey that certain embodiments include, while other embodiments do not include, certain features, elements or steps. Thus, such conditional language is not generally intended to imply that features, elements or steps are in any way required for one or more embodiments or that one or more embodiments necessarily include logic for deciding, with or without author input or prompting, whether these features, elements and steps are included or are to be performed in any particular embodiment.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited. In some contexts, description of an operation or event as occurring or being performed "based on," or "based at least in part on," a stated event or condition can be interpreted as being triggered by or performed in response to the stated event or condition.

With respect to the various methods and processes disclosed herein, although certain orders of operations or steps are illustrated and described, it should be understood that the various steps and operations shown and described may be performed in any suitable or desirable temporal order. Furthermore, any of the illustrated or described operations or steps may be omitted from any given method or process, and the illustrated or described methods and processes may include additional operations or steps not explicitly illustrated or described.

It should be appreciated that in the above description of embodiments, various features are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated or described in a particular embodiment herein can be applied to or used with any other embodiment. Further, no component, feature, step, or group of components, features, or steps are necessary or indispensable for each embodiment. Thus, it is intended that the scope of the disclosure herein and claimed below should not be limited by the particular embodiments described above but should be determined only by a fair reading of the claims that follow.

Unless the context clearly requires otherwise, throughout the description and the claims, the terms "comprise," "comprising," "have," "having," "include," "including," and the like are to be construed in an open and inclusive sense, as opposed to a closed, exclusive, or exhaustive sense; that is to say, in the sense of "including, but not limited to."

The word "coupled", as generally used herein, refers to two or more elements that may be physically, mechanically, or electrically connected or otherwise associated, whether directly or indirectly (e.g., via one or more intermediate elements, components, or devices. Additionally, the words "herein," "above," "below," and words of similar import, when used in this application, shall refer to this application as a whole, and not to any particular portions of the present disclosure. Where the context permits, words in present disclosure using the singular or plural number may also include the plural or singular number, respectively.

As may be used herein, the terms "substantially" and "approximately" provide an industry-accepted tolerance for its corresponding term or relativity between items. For some industries, an industry-accepted tolerance is less than one percent, while for other industries, the industry-accepted tolerance may be 10 percent or more. Other examples of industry-accepted tolerances range from less than one percent to fifty percent. Industry-accepted tolerances may correspond to, but are not limited to, component values, integrated circuit process variations, temperature variations, rise and fall times, thermal noise, dimensions, signaling errors, dropped packets, temperatures, pressures, material compositions, or performance metrics. Within an industry, tolerance variances of accepted tolerances may be more or less than a percentage level (e.g., dimension tolerance of less than approximately +/- 1%). Some relativity between items may range from a difference of less than a percentage level to a few percent. Other relativity between items may range from a difference of a few percent to magnitude of differences.

One or more embodiments may have been described above with the aid of method steps illustrating performance of specified functions and relationships thereof. The boundaries and sequence of these functional building blocks or method steps have been arbitrarily defined herein for convenience of description. Alternate boundaries and sequences can be defined so long as the specified functions and relationships are appropriately performed. Any such alternate boundaries or sequences are thus within the scope and spirit of the claims. Further, the boundaries of these functional building blocks may have been arbitrarily defined for convenience of description. Alternate boundaries could be defined as long as the certain significant functions are appropriately performed. Similarly, flow diagram blocks may also have been arbitrarily defined herein to illustrate certain significant functionality.

To the extent used, the flow diagram block boundaries and sequence could have been defined otherwise and still perform the certain significant functionality. Such alternate definitions of both functional building blocks and flow diagram blocks and sequences are thus within the scope and spirit of the disclosure. One of average skill in the art will also recognize that the functional building blocks, and other illustrative blocks, modules and components herein, can be implemented, in some examples, as illustrated or by discrete components, application specific integrated circuits, processors executing appropriate software and the like or any combination thereof.

In view of the many possible embodiments to which the principles of the disclosure may be applied, it should be recognized that the illustrated embodiments are only examples of the subject matter and should not be taken as limiting the scope of the disclosure.

## Claims

1.  A device (10) for providing fluid flow between fluid vessels (12, 14) of a patient, comprising:

    a conduit structure (18) configured to be implanted within a first fluid vessel (14), the conduit structure (18) comprising an outer wall (26) defining a conduit lumen (32) extending between a conduit inlet (28, 34) and a conduit outlet (30, 36), the conduit structure (18) comprising a constricted portion (38) of the conduit lumen (32) positioned between the conduit inlet (28, 34) and the conduit outlet (30, 36), the constricted portion (38) comprising a constricted lumen diameter (40b) that is less than a diameter (40a) of, both, the conduit inlet (28, 34) and the conduit outlet (30, 36); and
    a shunt structure (16, 24) extending in a direction substantially perpendicular to a longitudinal axis of the conduit lumen (32), such as to be configured to be at least partially implanted within a second fluid vessel (12) that is adjacent to the first fluid vessel (14) through a passage (222) obtained by puncturing through a wall of the first fluid vessel and a wall of the second fluid vessel, the shunt structure configured to attach to the constricted portion (38) of the conduit structure (18), the shunt structure (16, 24) comprising a shunt lumen (46) that is configured to be in fluid communication with the conduit lumen (32).

2. The device (10, 200) of claim 1, wherein the shunt lumen (46) comprises a shunt lumen diameter (48) that is no greater than the constricted lumen diameter (40b).

3. The device of claim 2, wherein the shunt lumen diameter (48) is 0.8 or less of the constricted lumen diameter (40b).

4. The device (10, 200) of any of claims 1 to 3, wherein the shunt structure comprises a shunt anchor (24) is configured to be secured to at least a portion of the second fluid vessel (12).

5. The device of claim 4, wherein the shunt anchor (24) comprises a stent adapted to be radially deployed within the second fluid vessel (12) and placed into contact with the wall of the second fluid vessel (12), in particular wherein the shunt anchor (24) comprises a disk-shaped form.

6. The device (10, 200) of any of claims 1 to 5, wherein the conduit structure (18) comprises a self-expanding stent frame configured to expand radially to engage a wall of the first fluid vessel (14).

7. The device (10, 200) of claim 6, wherein the self-expanding stent frame has a delivery configuration and an expanded configuration, the self-expanding stent frame being biased toward the expanded configuration.

8. The device (10, 200) of any of claims 1 to 7, wherein the conduit structure (18) and the shunt structure (16, 24) each comprise a self-expanding structure.

9. The device (10, 200) of any of claims 1 to 8, wherein the conduit structure (18) and the shunt structure (16, 24) each comprise memory material and are biased toward an expanded configuration.

10. The device (10, 200) of any of claims 1 to 9, wherein the conduit structure (18) comprises a bifurcation between the constricted portion (38) and the conduit outlet (30, 36), and the conduit structure (18) further comprises an outlet downstream of the bifurcation.

11. The device (10, 200) of any of claims 1 to 10, wherein the constricted lumen diameter (40b) is less than 0.8 of the diameter of the conduit inlet (28, 34).

12. A system for providing fluid flow between fluid vessels of a patient, comprising:

a device (10, 200) according to anyone of the preceding claims 1 to 11; and
a delivery catheter (164, 226) to removably receive the conduit structure (18) and the shunt structure (16, 24), the delivery catheter (164, 226) configured to hold at least one of the conduit structure (18) or the shunt structure (16, 24) in a compressed delivery configuration.

13. The system of claim 12, wherein the conduit structure (18) is self-expanding, and the delivery catheter (164, 226) comprises a sheath configured to slidingly receive the conduit structure (18).

14. The system of claim 12, wherein the delivery catheter (164, 226) comprises a radially expandable balloon (174) configured to deploy the conduit structure (18) within the first fluid vessel (14).

15. The system of claim 12, wherein the delivery catheter (164, 226) is configured to deploy the shunt structure (16, 24) between the first fluid vessel (14) and the second fluid vessel (12).

**Patentansprüche**

1. Vorrichtung (10) zur Bereitstellung eines Fluidflusses zwischen Fluidgefäßen (12, 14) eines Patienten, umfassend:

eine Leitungsstruktur (18), die dazu konfiguriert ist, in ein erstes Fluidgefäß (14) implantiert zu werden, wobei die Leitungsstruktur (18) eine Außenwand (26) umfasst, die ein Leitungslumen (32) definiert, das sich zwischen einem Leitungseinlass (28, 34) und einem Leitungsauslass (30, 36) erstreckt, wobei die Leitungsstruktur (18) einen verengten Abschnitt (38) des Leitungslumens (32) umfasst, der zwischen dem Leitungseinlass (28, 34) und dem Leitungsauslass (30, 36) angeordnet ist, wobei der verengte Abschnitt (38) einen verengten Lumendurchmesser (40b) umfasst, der kleiner ist als ein Durchmesser (40a) sowohl des Leitungseinlasses (28, 34) als auch des Leitungsauslasses (30, 36); und

eine Shunt-Struktur (16, 24), die sich in einer Richtung im Wesentlichen senkrecht zu einer Längsachse des Leitungslumens (32) erstreckt, so dass sie dazu konfiguriert ist, zumindest teilweise innerhalb eines zweiten Fluidgefäßes (12) implantiert zu werden, das zu dem ersten Fluidgefäß (14) benachbart ist, und zwar durch einen Durchgang (222), der durch Punktion durch eine Wand des ersten Fluidgefäßes und eine Wand des zweiten Fluidgefäßes erhalten wird, wobei die Shunt-Struktur dazu konfiguriert ist, an dem verengten Abschnitt (38) der Leitungsstruktur (18) angebracht zu werden, wobei die Shunt-Struktur (16, 24) ein Shunt-Lumen (46) umfasst, das dazu konfiguriert ist, in Fluidverbindung mit dem Leitungslumen (32) zu stehen.

2. Vorrichtung (10, 200) nach Anspruch 1, wobei das Shunt-Lumen (46) einen Shunt-Lumendurchmesser (48) umfasst, der nicht größer ist als der verengte Lumendurchmesser (40b).

3. Vorrichtung nach Anspruch 2, wobei der Durchmesser des Shunt-Lumens (48) 0,8 oder weniger vom verengten Lumendurchmesser (40b) beträgt.

4. Vorrichtung (10, 200) nach einem der Ansprüche 1 bis 3, wobei die Shunt-Struktur einen Shunt-Anker (24) umfasst, der dazu konfiguriert ist, an mindestens einem Abschnitt des zweiten Fluidgefäßes (12) befestigt zu werden.

5. Vorrichtung nach Anspruch 4, wobei der Shunt-Anker (24) einen Stent umfasst, der dazu eingerichtet ist, innerhalb des zweiten Fluidgefäßes (12) radial entfaltet und in Kontakt mit der Wand des zweiten Fluidgefäßes (12) platziert zu werden, insbesondere wobei der Shunt-Anker (24) eine scheibenförmige Form aufweist.

6. Vorrichtung (10, 200) nach einem der Ansprüche 1 bis 5, wobei die Leitungsstruktur (18) einen selbstexpandierenden Stentrahmen umfasst, der dazu konfiguriert ist, radial zu expandieren, um mit einer Wand des ersten Fluidgefäßes (14) in Eingriff zu kommen.

7. Vorrichtung (10, 200) nach Anspruch 6, wobei der selbstexpandierende Stentrahmen eine Zuführkonfiguration und eine expandierte Konfiguration aufweist, wobei der selbstexpandierende Stentrahmen in Richtung der expandierten Konfiguration vorgespannt ist.

8. Vorrichtung (10, 200) nach einem der Ansprüche 1 bis 7, wobei die Leitungsstruktur (18) und die Shunt-Struktur (16, 24) jeweils eine selbstexpandierende Struktur umfassen.

9. Vorrichtung (10, 200) nach einem der Ansprüche 1 bis 8, wobei die Leitungsstruktur (18) und die Shunt-Struktur (16, 24) jeweils Gedächtnismaterial umfassen und in Richtung einer expandierten Konfiguration vorgespannt sind.

10. Vorrichtung (10, 200) nach einem der Ansprüche 1 bis 9, wobei die Leitungsstruktur (18) eine Verzweigung zwischen dem verengten Abschnitt (38) und dem Leitungsauslass (30, 36) umfasst, und die Leitungsstruktur (18) ferner einen Auslass stromabwärts der Verzweigung umfasst.

11. Vorrichtung (10, 200) nach einem der Ansprüche 1 bis 10, wobei der Durchmesser des verengten Lumens (40b) weniger als 0,8 vom Durchmesser des Leitungseinlasses (28, 34) beträgt.

12. System zur Bereitstellung eines Fluidflusses zwischen Fluidgefäßen eines Patienten, umfassend:

eine Vorrichtung (10, 200) nach einem der vorangehenden Ansprüche 1 bis 11; und
einen Zuführkatheter (164, 226) zum entnehmbaren Aufnehmen der Leitungsstruktur (18) und der Shunt-Struktur (16, 24), wobei der Zuführkatheter (164, 226) dazu konfiguriert ist, mindestens eine von der Leitungsstruktur (18) oder der Shunt-Struktur (16, 24) in einer komprimierten Zuführkonfiguration zu halten.

13. System nach Anspruch 12, wobei die Leitungsstruktur (18) selbstexpandierend ist und der Zuführkatheter (164, 226) eine Hülle umfasst, die dazu konfiguriert ist, die Leitungsstruktur (18) gleitend aufzunehmen.

14. System nach Anspruch 12, wobei der Zuführkatheter (164, 226) einen radial expandierbaren Ballon (174) umfasst, der dazu konfiguriert ist, die Leitungsstruktur (18) innerhalb des ersten Fluidgefäßes (14) zu entfalten.

15. System nach Anspruch 12, wobei der Zuführkatheter (164, 226) dazu konfiguriert ist, die Shunt-Struktur (16, 24) zwischen dem ersten Fluidgefäß (14) und dem zweiten Fluidgefäß (12) zu entfalten.

**Revendications**

1. Dispositif (10) destiné à assurer un écoulement de fluide entre des vaisseaux sanguins (12, 14) d'un patient, comprenant :

   une structure de conduit (18) conçue pour être implantée à l'intérieur d'un premier vaisseau sanguin (14), la structure de conduit (18) comprenant une paroi externe (26) définissant une lumière de conduit (32) s'étendant entre une entrée de conduit (28, 34) et une sortie de conduit (30, 36), la structure de conduit (18) comprenant une partie rétrécie (38) de la lumière de conduit (32) positionnée entre l'entrée de conduit (28, 34) et la sortie de conduit (30, 36), la partie rétrécie (38) comprenant un diamètre (40b) de lumière rétrécie qui est inférieur à un diamètre (40a), à la fois, de l'entrée de conduit (28, 34) et de la sortie de conduit (30, 36) ; et
   une structure de dérivation (16, 24) s'étendant dans une direction sensiblement perpendiculaire à un axe longitudinal de la lumière de conduit (32), de manière à être conçue pour être au moins partiellement implantée à l'intérieur d'un second vaisseau sanguin (12) qui est adjacent au premier vaisseau sanguin (14) à travers un passage (222) obtenu par perforation à travers une paroi du premier vaisseau sanguin et une paroi du second vaisseau sanguin, la structure de dérivation étant conçue pour s'attacher à la partie rétrécie (38) de la structure de conduit (18), la structure de dérivation (16, 24) comprenant une lumière de dérivation (46) qui est conçue pour être en communication fluidique avec la lumière de conduit (32).

2. Dispositif (10, 200) selon la revendication 1, la lumière de dérivation (46) comprenant un diamètre (48) de lumière de dérivation qui n'est pas supérieur au diamètre (40b) de lumière rétrécie.

3. Dispositif selon la revendication 2, le diamètre (48) de lumière de dérivation étant inférieur ou égal à 0,8 fois le diamètre (40b) de lumière rétrécie.

4. Dispositif (10, 200) selon l'une quelconque des revendications 1 à 3, la structure de dérivation comprenant un ancrage de dérivation (24) conçu pour être fixé à au moins une partie du second vaisseau sanguin (12).

5. Dispositif selon la revendication 4, l'ancrage de dérivation (24) comprenant une endoprothèse conçue pour être déployée radialement à l'intérieur du second vaisseau sanguin (12) et placée en contact avec la paroi du second vaisseau sanguin (12), en particulier l'ancrage de dérivation (24) comprenant une forme façonnée en disque.

6. Dispositif (10, 200) selon l'une quelconque des revendications 1 à 5, la structure de conduit (18) comprenant un cadre d'endoprothèse autodéployable conçu pour se déployer radialement afin de venir en prise avec une paroi du premier vaisseau sanguin (14).

7. Dispositif (10, 200) selon la revendication 6, le cadre d'endoprothèse autodéployable présentant une configuration de pose et une configuration déployée, le cadre d'endoprothèse autodéployable étant sollicité vers la configuration déployée.

8. Dispositif (10, 200) selon l'une quelconque des revendications 1 à 7, la structure de conduit (18) et la structure de dérivation (16, 24) comprenant chacune une structure autodéployable.

9. Dispositif (10, 200) selon l'une quelconque des revendications 1 à 8, la structure de conduit (18) et la structure de dérivation (16, 24) comprenant chacune un matériau à mémoire et étant sollicitées vers une configuration déployée.

10. Dispositif (10, 200) selon l'une quelconque des revendications 1 à 9, la structure de conduit (18) comprenant une bifurcation entre la partie rétrécie (38) et la sortie de conduit (30, 36) et la structure de conduit (18) comprenant en outre une sortie en aval de la bifurcation.

11. Dispositif (10, 200) selon l'une quelconque des revendications 1 à 10, le diamètre (40b) de lumière rétrécie étant inférieur à 0,8 fois le diamètre de l'entrée de conduit (28, 34).

**12.** Système destiné à assurer un écoulement de fluide entre des vaisseaux sanguins d'un patient, comprenant :

un dispositif (10, 200) selon l'une quelconque des revendications précédentes 1 à 11 ; et
un cathéter de pose (164, 226) destiné à recevoir de manière amovible la structure de conduit (18) et la structure de dérivation (16, 24), le cathéter de pose (164, 226) étant conçu pour maintenir au moins une structure parmi la structure de conduit (18) ou la structure de dérivation (16, 24) dans une configuration de pose comprimée.

**13.** Système selon la revendication 12, la structure de conduit (18) étant autodéployable et le cathéter de pose (164, 226) comprenant une gaine conçue pour recevoir de manière coulissante la structure de conduit (18).

**14.** Système selon la revendication 12, le cathéter de pose (164, 226) comprenant un ballonnet (174) radialement extensible conçu pour déployer la structure de conduit (18) à l'intérieur du premier vaisseau sanguin (14).

**15.** Système selon la revendication 12, le cathéter de pose (164, 226) étant conçu pour déployer la structure de dérivation (16, 24) entre le premier vaisseau sanguin (14) et le second vaisseau sanguin (12).

*FIG. 1*

*FIG. 2A*

*FIG. 2B*

FIG. 2C

FIG. 2D

*FIG. 3*

FIG. 4-1

FIG. 4-2

FIG. 5

*FIG. 6*

EP 3 955 832 B1

110

112

116a                    114                    116b

FIG. 7A

112

110

116a          114          116b

FIG. 7B

116b

110

116a          114

FIG. 7C

FIG. 8C

FIG. 8B

FIG. 8A

28

FIG. 9

FIG. 10

FIG. 11A

FIG. 11B

*FIG. 11C*

*FIG. 11D*

EP 3 955 832 B1

FIG. 12A

FIG. 12B

FIG. 12C

FIG. 12D

*300*

| SECURE A SHUNT STRUCTURE AND/OR CONDUIT STRUCTURE TO A DELIVERY CATHETER | *302* |

↓

| ADVANCE THE DELIVERY CATHETER TO A TARGET LOCATION | *304* |

↓

| CREATE A PASSAGE BETWEEN THE FIRST FLUID VESSEL AND THE SECOND FLUID VESSEL | *306* |

↓

| ADVANCE THE DELIVERY CATHETER INTO ANATOMY BETWEEN THE FIRST FLUID VESSEL AND THE SECOND FLUID VESSEL | *308* |

↓

| DEPLOY THE CONDUIT STRUCTURE WITHIN THE SECOND FLUID VESSEL | *310* |

↓

| DEPLOY THE SHUNT STRUCTURE IN THE ANATOMY BETWEEN THE FIRST FLUID VESSEL AND THE SECOND FLUID VESSEL | *312* |

↓

| DEPLOY AN ANCHOR WITHIN THE FIRST FLUID VESSEL | *314* |

↓

| ADJUST A DIAMETER OF THE CONDUIT STRUCTURE | *316* |

*FIG. 13*

FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003024527 A1
- US 2006287704 A1
- WO 2015013344 A2